# EUROPÄISCHE PATENTSCHRIFT

(11) **EP 3 153 174 B1**
(45) Veröffentlichungstag und Bekanntmachung des Hinweises auf die Patenterteilung: **20.02.2019**
(21) Anmeldenummer: 15188428.5
(22) Anmeldetag: 05.10.2015
(51) Int. Cl.: A61K 36/84, A61K 36/185, A61K 36/31, A61P 25/20

(54) **NATÜRLICHE ZUSAMMENSETZUNGEN ZUR BEHANDLUNG VON SCHLAFSTÖRUNGEN**
NATURAL COMPOSITIONS FOR THE TREATMENT OF INSOMNIA
COMPOSITIONS NATURELLES DESTINEES AU TRAITEMENT DES TROUBLES DU SOMMEIL

(43) Veröffentlichungstag der Anmeldung: 12.04.2017
(73) Patentinhaber: BioActive Food GmbH, 23795 Bad Segeberg (DE)
(72) Erfinder: VOLLERT, Henning, 23795 Bad Segeberg (DE); DIMPFEL,Wilfried, 35578 Wetzlar (DE)
(74) Vertreter: Uexküll & Stolberg

(56) Entgegenhaltungen:
- EP-A1- 2 522 346
- DATABASE WPI Week 201424 Thomson Scientific, London, GB; AN 2014-B44584 XP002755230, & CN 103 393 162 A (MAANSHAN HUANGCHI FOOD GROUP CO LTD) 20. November 2013 (2013-11-20)
- DATABASE WPI Week 201336 Thomson Scientific, London, GB; AN 2013-G65179 XP002755231, & CN 102 813 897 A (MA X) 12. Dezember 2012 (2012-12-12)
- DATABASE WPI Week 201422 Thomson Scientific, London, GB; AN 2014-F32294 XP002755232, & CN 103 535 819 A (HEFEI KANGLING CURING TECHNOLOGY CO LTD) 29. Januar 2014 (2014-01-29)
- DATABASE WPI Week 200948 Thomson Scientific, London, GB; AN 2009-L49070 XP002755233, & JP 2009 155341 A (FANCL CORP) 16. Juli 2009 (2009-07-16)
- DATABASE WPI Week 200416 Thomson Scientific, London, GB; AN 2004-162171 XP002755234, & JP 2004 043378 A (FANCL CORP) 12. Februar 2004 (2004-02-12)

## Beschreibung

Die vorliegende Erfindung betrifft eine neuartige und hochwirksame Zusammensetzung, die als Beruhigungsmittel zur Behandlung oder Vorbeugung von Schlafstörungen, Aufmerksamkeitsdefizit-/Hyperaktivitätsstörungen (ADHS) und Zuständen erhöhter Nervosität verwendet werden kann. Die Zusammensetzung lässt sich ferner zur Verbesserung der kognitiven Fähigkeiten und insbesondere zur Erhöhung der Konzentrationsfähigkeit verwenden. Die wirksamen Inhaltsstoffe der Zusammensetzung sind ausschließlich natürlichen Ursprungs und daher gut verträglich. Die Zusammensetzung umfasst (a) mindestens einen Extrakt aus einer Pflanze der Gattung Brassica oder Teile einer Pflanze der Gattung Brassica, wobei die Pflanze der Gattung Brassica Grünkohl ist, und mindestens einen der beiden folgenden Bestandteile: (b1) mindestens einen Extrakt aus einer Pflanze der Gattung Valeriana oder Teile einer Pflanze der Gattung Valeriana, und (b2) mindestens einen Extrakt aus einer Pflanze der Gattung Humulus oder Teile einer Pflanze der Gattung Humulus. Die Erfindung betrifft ferner ein Arzneimittel, Nahrungsergänzungsmittel oder Lebensmittel, das die erfindungsgemäße Zusammensetzung umfasst.

### GEBIET DER ERFINDUNG

Nach einer Untersuchung des Robert-Koch-Instituts leiden etwa 25% der Bevölkerung in Deutschland an Schlafstörungen. Etwa 11% der Bevölkerung erleben ihren Schlaf als "häufig nicht erholsam". Die Folgen von Schlafstörungen äußern sich bei Betroffenen durch erhöhte Tagesmüdigkeit, erhöhte Vergesslichkeit, Antriebsschwäche und eine verringerte Merk- und Konzentrationsfähigkeit. Obwohl Schlafstörungen allgemein nicht als schwerwiegende Störung angesehen werden, verursachen die damit verbundenen Einschränkungen der Leistungsfähigkeit jedes Jahr Schäden in beträchtlicher Höhe, z.B. durch Fehler im beruflichen Umfeld oder im Straßenverkehr.

Die Ursachen von Schlafstörungen können vielfältig sein. Neben organischen Beschwerden, wie z.B. Durchblutungsstörungen, Bluthochdruck, Magen-Darm-Beschwerden oder Funktionsstörungen der Schilddrüse, können auch Störungen im familiären oder beruflichen Umfeld, wie z.B. Stress, Erschöpfungszustände, Versagensängste, als Ursachen für Schlafstörungen in Betracht kommen. Der Ausbau schlafmedizinischer Zentren in Krankenhäusern und Universitätskliniken hat die Diagnose von Schlafstörungen und die Identifizierung der entsprechenden Ursachen erheblich verbessert.

Allerdings sind die Behandlungsmöglichkeiten auch weiterhin unzureichend. Neben der Befolgung bestimmter Verhaltensregeln, wie z.B. dem zeitgerechten Umgang mit dem Tag-Nacht-Rhythmus und der Vermeidung von potentiell schlafstörenden Genussmitteln wie Alkoholika, Tabak und Kaffee, kommen schlaffördernde Mittel gegenwärtig nur im geringen Umfang zum Einsatz. Pharmakologisch aktive Schlafmittel, wie Benzodiazepine, Chloralhydrat und Opipramol, werden aufgrund des überschaubaren Leidensdrucks und insbesondere wegen der teilweise erheblichen Nebenwirkungen von Betroffenen abgelehnt. So ist beispielsweise bekannt, dass die Einnahme von Opipramol oftmals mit Müdigkeit, Schwindel und schwerwiegenden gastrointestinalen Nebenwirkungen einhergehen kann.

Vor diesem Hintergrund kommt der Behandlung von Schlafstörungen mit natürlichen, insbesondere pflanzlichen, Mitteln eine besondere Bedeutung zu. Präparate, die Hopfen oder Baldrianextrakt enthalten, werden in großem Umfang bei der Behandlung von Schlafstörungen eingesetzt (z.B. Baldiparan®). Allerdings weisen natürliche schlaffördernde Substanzen in der Regel eine unzureichende Wirkung auf. So ist z.B. die beruhigende und schlaffördernde Wirkung von Baldrian wissenschaftlich belegt, wenn auch die Wirkungen bei vielen Betroffenen nur sehr gering bis kaum nachweisbar sind.

Es besteht daher ein Bedürfnis nach neuen Substanzen und Präparaten, die sich zur Behandlung von Schlafstörungen eignen. Die Präparate und Zusammensetzungen sollten dabei möglichst natürlichen Ursprungs und ohne Nebenwirkungen sein, um eine hohe Akzeptanz bei den Betroffenen zu erreichen. Die vorliegende Erfindung trägt diesem Bedürfnis Rechnung und stellt Zusammensetzungen bereit, deren schlaffördernd und/oder beruhigend wirksamen Bestandteile pflanzlichen Ursprungs und frei von Nebenwirkungen sind.

### BESCHREIBUNG DER ERFINDUNG

Es wurde im Rahmen der vorliegenden Erfindung überraschend festgestellt, dass die beruhigende und/oder schlaffördernde Wirkung eines Extrakts aus einer Pflanze der Gattung Valeriana oder der Gattung Humulus durch einen Grünkohl-Extrakt erheblich verstärkt werden kann. Eine schlaffördernde Wirkung von Grünkohlextrakt wurde bislang nicht beschrieben. Wie in den anliegenden Beispielen gezeigt wird, beruht die Verstärkung der Wirkung auf einem synergistischen Effekt und ist durch eine Addition der Wirkungen der Extrakte nicht zu erklären. Die gewünschte beruhigende und/oder schlaffördernde Wirkung lässt sich erfindungsgemäß ferner auch durch Kombination von Teilen der besagten Pflanzen (d.h. Pflanzenteilen) erreichen, z.B. durch Kombination von Baldrianwurzeln und/oder Hopfenblüten mit Grünkohlblättern in einer essbaren Zusammensetzung.

Somit stellt die vorliegende Erfindung in einem ersten Aspekt eine Zusammensetzung mit beruhigender und/oder schlaffördernder Wirkung bereit, die folgenden Bestandteil umfasst:
(a) mindestens einen Extrakt aus einer Pflanze der Gattung Brassica oder Teile einer Pflanze der Gattung Brassica, wobei die Pflanze der Gattung Brassica Grünkohl ist.

Darüber hinaus enthält die Zusammensetzung mindestens einen der beiden nachfolgenden Bestandteile (b1) und (b2):
(b1) mindestens einen Extrakt aus einer Pflanze der Gattung Valeriana oder Teile einer Pflanze der Gattung Valeriana;
(b2) mindestens einen Extrakt aus einer Pflanze der Gattung Humulus oder Teile einer Pflanze der Gattung Humulus.

Als ersten wirksamen Bestandteil umfasst die erfindungsgemäße Zusammensetzung einen oder mehrere, d.h. 2, 3, 4, 5 oder mehr, Extrakte aus einer Pflanze der Gattung Brassica oder Teile einer Pflanze dieser Gattung, wobei die Pflanze der Gattung Brassica Grünkohl ist. Bei der Gattung Brassica handelt es sich um eine pflanzliche Gattung innerhalb der Familie der Kreuzblütengewächse (Brassicaceae). Bei der Spezies Brassica oleracea handelt es sich um eine formenreiche Pflanzenart innerhalb der Gattung Brassica. Die Art umfasst zahlreiche Sorten, zu denen u.a. Brokkoli (Brassica oleracea var. silvestris L.), Rotkohl (Brassica oleracea var. capitata f. rubra L.), Weißkohl (Brassica oleracea var. capitata f. alba), Wirsing (Brassica oleracea L. convar. capitata (L.) Alef. var. sabauda L.), Chinakohl (Brassica rapa ssp. pekinensis) und Grünkohl (Brassica oleracea convar. acephala var. sabellica) zählen. Gemäß einer besonders bevorzugten Ausführungsform enthält die erfindungsgemäße Zusammensetzung einen oder mehrere Extrakte, die ausgehend von vollständigen Grünkohl-Pflanzen oder Teilen derselben hergestellt wurden. Gemäß der vorliegenden Erfindung ist der Brassica-Extrakt oder die Brassica-Extrakte, die für die Herstellung der erfindungsgemäßen Zusammensetzung verwendet werden, aus Grünkohl hergestellt, insbesondere aus Grünkohlblättern. Sofern die Zusammensetzung Pflanzenteile von Brassica enthält, ist es bevorzugt, dass es sich bei den Pflanzenteilen um Grünkohlblätter handelt.

Als weiteren wirksamen Bestandteil kann die erfindungsgemäße Zusammensetzung einen bzw. mehrere Extrakte aus einer Pflanze der Gattung Valeriana umfassen. Neben den Extrakten oder anstelle der Extrakte können auch Teile einer Pflanze der Gattung Valeriana in die Zusammensetzungen eingebracht werden. Bei der Gattung Valeriana handelt es sich um eine Pflanzengattung aus der Unterfamilie der Baldriangewächse (Valerianoideae). Sie umfasst etwa 150 bis 250 Pflanzenarten. Gemäß der vorliegenden Erfindung können Pflanzenteile oder Extrakte von allen bekannten Arten zur Herstellung der erfindungsgemäßen Zusammensetzung verwendet werden. Vorzugsweise handelt es sich bei den Extrakten um solche, die aus den Arten V. officinalis (echter Baldrian), V. altaica, V. amurensis, V. arborea, V. arizonica, V. barbulata, V. californica, V. capitata, V. celtica L., V. columbiana, V. dioica L., V. elongata, V. kassarica, V. montana L., V. occidentalis, V. officinalis, V. procurrens, V. pyrenaica L., V. saliunca, V. saxatilis L., V. tripteris L., V. wallrothii oder V. supina erhalten wurden. Extrakte aus V. officinalis sind besonders bevorzugt. Sofern die Zusammensetzung Pflanzenteile von Valeriana enthält, ist es bevorzugt, dass es sich bei den Pflanzenteilen um Wurzeln oder Teile der Wurzeln der Pflanze handelt. Wurzeln oder Wurzel-Teile von V. officinalis sind besonders bevorzugt.

Neben den von Valeriana erhaltenen Bestandteilen oder anstelle der von Valeriana erhaltenen Bestandteile kann die erfindungsgemäße Zusammensetzung einen Extrakt bzw. mehrere Extrakte aus einer Pflanze der Gattung Humulus oder Teile einer Pflanze der Gattung Humulus enthalten. Die Gattung Humulus (Hopfen) bezeichnet eine Pflanzengattung in der Familie der Hanfgewächse. Es können Pflanzenteile oder Extrakte von allen bekannten Humulus-Arten zur Herstellung der erfindungsgemäßen Zusammensetzung verwendet werden. Vorzugsweise handelt es sich bei den Extrakten oder Pflanzenteilen um solche, die von den Arten H. lupulus L. (echter Hopfen), H. scandens (Lour.) und H. yunnanensis Hu erhalten wurden. Sofern die Zusammensetzung Pflanzenteile von Humulus enthält, ist es bevorzugt, dass es sich bei den Pflanzenteilen um die Blüten oder Teile der Blüten der Pflanze handelt, vorzugsweise um weibliche Blüten oder Teile von weiblichen Blüten. Blüten oder Blütenteile von H. lupulus L. sind besonders bevorzugt.

Es ist erfindungsgemäß besonders bevorzugt, dass die Zusammensetzung der vorliegenden Erfindung die wirksamen pflanzlichen Bestandteile in Form von Extrakten enthält. Wie im Rahmen der vorliegenden Erfindung verwendet, bezeichnet der Begriff "Extrakt" ein Stoffgemisch, das durch Aufschluss von Zellen einer Pflanze und Gewinnung des Zellsafts erhalten wird. Folglich bezeichnet der Begriff "Brassica-Extrakt" vorliegend ein Stoffgemisch, das durch Aufschluss von Zellen einer Pflanze der Gattung Brassica und Gewinnung des Zellsafts erhalten wird. Entsprechend bezeichnet der Begriff "Valeriana-Extrakt" vorliegend ein Stoffgemisch, das durch Aufschluss von Zellen einer Pflanze der Gattung Valeriana und Gewinnung des Zellsafts erhalten wird. Der Begriff "Humulus-Extrakt" bezieht sich vorliegend auf ein Stoffgemisch, das durch Aufschluss von Zellen einer Pflanze der Gattung Humulus und Gewinnung des Zellsafts erhalten wird.

Die zur Verwendung in den erfindungsgemäßen Zusammensetzungen vorgeschlagenen Extrakte lassen sich käuflich erwerben. Insbesondere Baldrian-Extrakte und Hopfen-Extrakte sind von mehreren Anbietern erhältlich. Darüber hinaus lassen sich Extrakte von Pflanzen der Gattungen Brassica, Valeriana und Humulus durch eine Vielzahl von im Stand der Technik bekannten und hinreichend beschriebenen Verfahren herstellen. Grundsätzlich ist jedes dieser Verfahren für die Herstellung der Extrakte geeignet.

In einem besonders bevorzugten Verfahren zur Herstellung eines Brassica-Extrakts wird zunächst Material einer Brassica-Pflanze, insbesondere Grünkohl, bereitgestellt. Als Ausgangsmaterial für das erfindungsgemäße Verfahren eignen sich Pflanzen in sämtlichen Entwicklungsstadien, vom Samen bis zur reifen Pflanze. Ferner eignen sich sämtliche Pflanzenteile, wie z.B. die Früchte, Stängel, Blätter, Rinde oder Wurzeln der Pflanze. Das Ausgangsmaterial kann eine Mischung aus verschiedenen Pflanzenteilen sein, oder nur bestimmte Pflanzenteile umfassen. Besonders bevorzugt ist die Herstellung eines Extraktes aus Blättern der Brassica-Pflanze. Das pflanzliche Material wird in der Regel zunächst zerkleinert. Das Zerkleinern kann durch einfaches Zerstückeln der jeweiligen Pflanzenteile mit herkömmlichen Cuttern erreicht werden. Es lassen sich grundsätzlich alle Pflanzenteile als Ausgangspunkt für die Extrakte verwenden. Bei der Herstellung von Brassica-Extrakten werden besonders gute Ergebnisse erzielt, wenn Blätter der Pflanzen verwendet werden. Daher werden die Extrakte aus einer Pflanze der Gattung Brassica gemäß einer bevorzugten Ausführungsform der Erfindung aus den Blättern der jeweiligen Brassica-Pflanze hergestellt. Vor der Zerkleinerung kann das pflanzliche Material bei Temperaturen zwischen 45°C und 100°C blanchiert werden, um bakterielle Verunreinigungen zu beseitigen und die Qualität des Aufschlusses zu verbessern. Des Weiteren wird durch das Blanchieren die Aktivität von Enzymen wie Hydrolasen, Lipasen und Oxidasen reduziert und somit die Stabilität und Qualität des Pflanzenmaterials erhöht.

In einem nächsten Schritt wird das pflanzliche Material aufgeschlossen, d.h. die zellulären Strukturen des Materials werden zerstört, sodass der Inhalt der Zellen freigesetzt wird. Der Aufschluss kann durch herkömmliche Verfahren zum Aufschluss pflanzlicher Zellen erreicht werden, beispielsweise durch wiederholtes Einfrieren und Auftauen, oder durch geeignete Apparaturen, wie z.B. durch Homogenisatoren, Hochdruckhomogenisatoren oder Ultraschallhomogenisatoren. Auch Kolloidmühlen oder French-Pressen können für den Aufschluss verwendet werden.

Darüber hinaus kann der Zellaufschluss auch enzymatisch erzielt werden. Zu diesem Zweck wird das pflanzliche Material mit geeigneten Enzymen versetzt, die zu einer Zerstörung der strukturellen Bestandteile der Zellen führen. Es hat sich gezeigt, dass eine Inkubation des pflanzlichen Materials mit Pektinase, Kollagenase, Cellulase und/oder Hemicellulasen in dieser Phase des Verfahrens besonders geeignet ist, die Zellen wirkungsvoll aufzuschließen. Die Inkubationszeit kann zwischen 30 min und 24 h liegen, vorzugsweise zwischen 1 h und 6 h, stärker bevorzugt zwischen 90 min und 4 h, z.B. 2 h. Die Temperatur kann im Bereich von 20° und 60°C liegen, vorzugsweise zwischen 25°C und 45°C, z.B. bei ca. 37°C, und sie sollte vorzugsweise im Bereich des Temperaturoptimums des jeweils verwendeten Enzyms liegen. Ein geeigneter pH-Wert des Reaktionsansatzes kann unter Verwendung geeigneter Puffer, wie z.B. Phosphat-, Carbonat-, Natriumhydrogencarbonatpuffer und/oder geeigneter Säuren, wie z.B. Zitronensäure, Milchsäure oder Ascorbinsäure eingestellt werden. Ein pH Wert zwischen 4 und 6, z.B. pH 5, ist für den Aufschluss besonders geeignet.

Nach Aufschluss der Zellen kann der erhaltene Zellsaft direkt als Brassica-Extrakt zur Herstellung der erfindungsgemäßen Zusammensetzung verwendet werden. Es ist allerdings bevorzugt, den nach dem Aufschluss der Zellen erhaltenen Brassica-Pflanzensaft weiteren Reinigungsschritten zu unterziehen, die auf an eine Anreicherung der wirksamen Bestandteile des Extrakts gerichtet sind.

Beispielsweise kann das aus dem Zellaufschluss erhaltene Material einer Trocknung oder Gefriertrocknung mit anschließender Extraktion unterzogen werden. Hier kann beispielsweise zunächst eine Sprühtrocknung eingesetzt werden. Das getrocknete oder gefriergetrocknete pflanzliche Material kann anschließend mit einem wässrigen oder organischen Lösungsmittel extrahiert werden. Die Extraktion kann mit einem herkömmlichen Extraktionsmittel durchgeführt werden, z.B. mit Ethanol, Ethylacetat oder mit anderen organischen Lösungsmitteln. Auch eine Extraktion mit Gasen, wie z.B. mit Stickstoff ist möglich. Bei Verwendung eines flüssigen Lösungsmittels beträgt die Inkubationszeit 0,5 bis 24 h, vorzugsweise 2 bis 8 h. Bei Verwendung von Stickstoff erfolgt die Extraktion bei Temperaturen zwischen 4°C und 37°C in einem Zeitraum von 0,25 bis 3 h, vorzugsweise in einem Zeitraum von 20 und 60 Minuten.

Alternativ zu einer Trocknung/Extraktion kann der nach Aufschluss der Zellen erhaltene Pflanzensaft auch einer chromatographischen Aufreinigung unterzogen werden, beispielsweise unter Verwendung einer Ionenaustauschchromatographie oder einer Affinitätschromatographie. Die mit Ethanol oder Puffer eluierten Fraktionen können direkt verwendet oder eingetrocknet werden.

In einer bevorzugten Ausführungsform werden die Brassica-Extrakte bei ihrer Herstellung Schritten unterzogen, die zu einer Aufkonzentrierung der in den Pflanzen enthaltenen Polyphenole, Flavonoide und/oder Glycoside führen. Verfahren zur Aufkonzentrierung dieser Substanzen sind dem Fachmann hinreichend bekannt und werden z.B. in Will et al. (LWT - Food Science and Technology, 2006, 40(8):1344-1351) beschrieben. Eine Aufkonzentrierung der Polyphenole, Flavonoide und/oder Glycoside kann beispielsweise durch Verwendung geeigneter Säulenchromatographie-Verfahren erreicht werden. Zu diesem Zweck können verschiedene Adsorber-Säulen eingesetzt werden. Die Säulen werden mit dem Ausgangsextrakt beladen und durch Spülen mit geeigneten Lösungen von unerwünschten Bestandteilen befreit. Anschließend werden die an die Säule gebundenen Substanzen in aufkonzentrierter Form von der Säule eluiert. Zum Beispiel werden nach Beladen von Adsorber-Säulen (z.B. FPX 66™, Fa. Rohm & Haas) mit unkonzentriertem Extrakt durch Spülen mit demineralisiertem Wasser hydrophile Substanzen (Salze, Aminosäuren, Peptide, Zucker etc.) abgereichert. In dem mit 95% Ethanol eluiertem Extrakt sind anschließend Polyphenole angereichert.

In der oben am Beispiel von Brassica beschriebenen Weise lassen sich auch Valeriana- oder Humulus-Extrakte zur Verwendung in den erfindungsgemäßen Zusammensetzungen herstellen. Auch hier kommen grundsätzlich zunächst alle Teile der Pflanze für die Extraktherstellung in Betracht, z.B. Früchte, Stängel, Blätter, Rinde oder Wurzeln. Vorzugsweise handelt es sich bei dem Valeriana-Extrakt im Rahmen der vorliegenden Erfindung um einen Extrakt der Wurzel (Valerinanae radix). Die Wurzel von Valeriana-Pflanzen enthält verschiedene Inhaltsstoffe, von denen man annimmt, dass sie mit einer beruhigenden Wirkung assoziiert sind, z.B. Sesquiterpene, ätherische Öle, Iridoide, Alkaloide, Lignane, Flavonoide und andere. In einer besonders bevorzugten Ausführungsform handelt es sich bei dem Valeriana-Extrakt um einen Baldrianwurzel-Extrakt. Solche Extrakte sind im Handel unter der Bezeichnung "Baldrian-Extrakt" erhältlich und werden für die Behandlung von Schlafstörungen angeboten. In einer weiteren bevorzugten Ausführungsform handelt es sich bei dem Valeriana-Extrakt um ein Baldrian-Öl.

Bei dem erfindungsgemäß verwendeten Humulus-Extrakt kann es sich um einen Extrakt aus Hopfenblüten handeln. Die weibliche Blüte des Hopfens enthält verschiedene Inhaltsstoffe, von denen angenommen wird, dass sie eine beruhigende und schlaffördernde Wirkung ausüben, z.B. Bittersäuren, Humulon, Lupulon, ätherische Öle, Alkaloide, Flavonoide und andere. In einer besonders bevorzugten Ausführungsform handelt es sich bei dem Humulus-Extrakt um einen Extrakt aus Blüten der weiblichen Hopfenpflanze, z.B. um einen Hopfenzapfen-Extrakt. Solche Extrakte sind im Handel unter der Bezeichnung "Hopfen-Extrakt" oder "Hopfenzapfen-Extrakt" erhältlich und werden für die Behandlung von Schlafstörungen angeboten. In einer weiteren bevorzugten Ausführungsform handelt es sich bei dem Hopfen-Extrakt um ein Hopfen-Öl.

In einer Ausführungsform stellt die Erfindung eine Zusammensetzung bereit, die folgende Bestandteile enthält:
(a) einen Grünkohl-Extrakt; und
(b) einen Baldrian-Extrakt.

In einer weiteren Ausführungsform stellt die Erfindung eine Zusammensetzung bereit, die folgende Bestandteile enthält:
(a) einen Grünkohl-Extrakt; und
(b) einen Hopfen-Extrakt.

In einer noch weiteren Ausführungsform stellt die Erfindung eine Zusammensetzung bereit, die folgende Bestandteile enthält:
(a) einen Grünkohl-Extrakt;
(b) einen Hopfen-Extrakt; und
(c) einen Baldrian-Extrakt.

In einer noch weiteren Ausführungsform stellt die Erfindung eine Zusammensetzung bereit, die folgende Bestandteile enthält:
(a) einen Extrakt aus Grünkohlblättern; und
(b) einen Extrakt aus Baldrian-Wurzeln.

In einer noch weiteren Ausführungsform stellt die Erfindung eine Zusammensetzung bereit, die folgende Bestandteile enthält:
(a) einen Extrakt aus Grünkohlblättern; und
(b) einen Extrakt aus Hopfen-Blüten, vorzugsweise weiblichen Hopfen-Blüten.

In einer noch weiteren Ausführungsform stellt die Erfindung eine Zusammensetzung bereit, die folgende Bestandteile enthält:
(a) einen Extrakt aus Grünkohlblättern;
(b) einen Extrakt aus Hopfen-Blüten, vorzugsweise weiblichen Hopfen-Blüten; und
(c) einen Extrakt aus Baldrian-Wurzeln.

Die erfindungsgemäß vorgeschlagenen Zusammensetzungen lassen sich durch eine Vielzahl von im Stand der Technik bekannten Verfahren herstellen. So können beispielsweise die von der Zusammensetzung umfassten Extrakte aus den Pflanzen der Gattungen Brassica und Valeriana und/oder Humulus zunächst einzeln hergestellt und unmittelbar vor der Verwendung zu einer einheitlichen, die Extrakte umfassenden Zusammensetzung gemischt werden. Alternativ dazu können zunächst ein oder mehrere Brassica-Extrakte hergestellt und miteinander vermischt werden, und anschließend mit einem oder mehreren Valeriana-Extrakten und/oder mit einem oder mehreren Humulus-Extrakten oder mit einem Präparat, das einen Valeriana-Extrakt und/oder einen Humulus-Extrakt enthält, z.B. mit einem kommerziell erhältlichen Baldrianwurzel-Extrakt oder Hopfenblüten-Extrakt, versetzt werden.

Grundsätzlich ist es nicht von Bedeutung, in welcher Reihenfolge die Bestandteile der erfindungsgemäßen Zusammensetzungen miteinander gemischt werden, solange sie zeitnah an das zu behandelnde Subjekt verabreicht werden. Daher betrifft die vorliegende Erfindung auch die Verwendung von mindestens einem Grünkohl-Extrakt in Kombination mit mindestens einem Valeriana-Extrakt und/oder mindestens einem Humulus-Extrakt als Arznei- und/oder Beruhigungsmittel und/oder zur Behandlung und/oder zur Prophylaxe von Schlafstörungen, Aufmerksamkeitsdefizit-/Hyperaktivitätsstörung (ADHS) und Zuständen erhöhter Nervosität oder zur Verbesserung der kognitiven Fähigkeiten und insbesondere zur Erhöhung der Konzentrationsfähigkeit, wobei die Komponenten für die getrennte Verabreichung formuliert sind und nacheinander an das zu behandelnde Subjekt verabreicht werden. Entsprechendes gilt, wenn anstelle von einem oder mehreren Extrakten Teile der entsprechenden Pflanze verwendet werden.

In einer Ausführungsform liegen die wirksamen pflanzlichen Bestandteile als zerkleinerte oder unzerkleinerte Teile der entsprechenden Pflanzen vor. So kann die Zusammensetzung z.B. zerkleinerte oder unzerkleinerte Grünkohl-Blätter enthalten, die mit zerkleinerten oder unzerkleinerten Baldrianwurzeln und/oder zerkleinerten oder unzerkleinerten Hopfenblüten vermengt sind. Die Zusammensetzung kann beispielsweise vor der Aufnahme durch Zugabe zu einem geeigneten flüssigen Träger als Getränk formuliert werden. Alternativ können die Pflanzenteile auch durch Verwendung eines leistungsfähigen Mixer püriert werden. Auf diese Weise lässt sich die erfindungsgemäße Zusammensetzung als "Smoothie" formulieren. In einer Ausführungsform ist die erfindungsgemäße Zusammensetzung daher als Getränk formuliert.

Die in den erfindungsgemäßen Zusammensetzungen enthaltenen Extrakte können in einem beliebigen Mischungsverhältnis miteinander kombiniert werden. Vorzugsweise enthält die erfindungsgemäßen Zusammensetzung den Grünkohl-Extrakt und den Valeriana- bzw. Humulus-Extrakt im Verhältnis von ungefähr 20:1, 10:1, 5:1, 4:1, 3:1, 2:1, 1:1, 1:2, 1:3, 1:4, 1:5, 1:10 oder 1:20. Ein Mischungsverhältnis zwischen Grünkohl-Extrakt und Valeriana-Extrakt bzw. zwischen Grünkohl-Extrakt und Humulus-Extrakt von 1:1 ist besonders bevorzugt. Sofern die Zusammensetzung Extrakte von Grünkohl-, Valeriana- und Humulus-Pflanzen enthält, können die Extrakte ebenfalls in einem beliebigen Mischungsverhältnis kombiniert werden, z.B. im Verhältnis von etwa 20:1:1; 1:20:1; 1:1:20 sowie 1:1:1 und allen dazwischen liegenden Mischungsverhältnisse. Ein Mischungsverhältnis zwischen Grünkohl-, Humulus- und Valeriana-Extrakt von 1:1:1 ist besonders bevorzugt.

Da es sich bei den Grünkohl-Extrakten, Humulus-Extrakten und Valeriana-Extrakten um pflanzliche Extrakte handelt, können diese in verhältnismäßig hohen Dosen verwendet werden, ohne dass Nebenwirkungen zu erwarten wären. Grundsätzlich hängt die genaue zu verwendende Menge der Extrakte in der Zusammensetzung von der Art und der spezifischen Kombination der Extrakte sowie auch von der Herstellung der Extrakte ab. So ist für den Fachmann verständlich, dass Extrakte, die nach Aufschluss des Zellmaterials aufkonzentriert wurden (wie es oben beschrieben wurde), im Vergleich zu nicht aufkonzentrierten Extrakten in geringeren Mengen verwendet werden können. Der Fachmann wird ohne Weiteres in der Lage sein, geeignete Mengen der Extrakte zu kombinieren, um die gewünschte Wirkung zu erhalten.

Die bevorzugte Tagesmenge an Grünkohl-Extrakt wird bei oralen oder rektalen Verabreichungsformen etwa 50-1000 mg betragen, vorzugsweise etwa 75-800 mg, 100-700 mg, und besonders bevorzugt etwa 200-600 mg, z.B. etwa 500 mg. Bei parenteralen (zum Beispiel intravenösen, subkutanen, intramuskulären) Verabreichungsformen wird die bevorzugte Tagesmenge an Grünkohl-Extrakt etwa 50-500 mg betragen, vorzugsweise etwa 100-300 mg, z.B. etwa 250 mg. Die zu verabreichende Tagesmenge kann in einer Dosiseinheit oder in mehreren Dosiseinheiten, beispielsweise in 1, 2 oder 3 Dosiseinheiten verabreicht werden. In einer besonders bevorzugten Ausführungsform der Erfindung beziehen sich die obigen Mengenangaben auf einen Grünkohl-Extrakt.

Die bevorzugte Tagesmenge an Valeriana-Extrakt und/oder Humulus-Extrakt wird bei oralen, parenteralen oder rektalen Verabreichungsformen etwa 100-1500 mg betragen, vorzugsweise etwa 150-1000 mg, etwa 200-800 mg, und besonders bevorzugt etwa 250-700 mg, z.B. etwa 500 oder 600 mg. Die zu verabreichende Tagesmenge kann in einer Dosiseinheit oder in mehreren Dosiseinheiten, beispielsweise in 1, 2 oder 3 Dosiseinheiten verabreicht werden. In einer besonders bevorzugten Ausführungsform der Erfindung beziehen sich die obigen Mengenangaben auf einen Baldrianwurzel-Extrakt bzw. Hopfenzapfenextrakt.

Demgemäß umfassen die erfindungsgemäßen Zusammensetzungen vorzugsweise bezogen auf das Gesamtgewicht der Zusammensetzung:
- mindestens 1%, 2%, 3%, 4%, 5%, 6%, 7%, 8%, 9%, 10%, 12% 15%, 20%, 22% oder 25% oder mehr (Gew./Gew.) Grünkohl-Extrakt, z.B. Grünkohl-Extrakt, und
- mindestens 1%, 2%, 3%, 4%, 5%, 6%, 7%, 8%, 9%, 10%, 12% 15%, 20%, 22% oder 25% oder mehr(Gew./Gew.) Valeriana-Extrakt, z.B. Baldrian-Extrakt, und/oder
- mindestens 1%, 2%, 3%, 4%, 5%, 6%, 7%, 8%, 9%, 10%, 12% 15%, 20%, 22% oder 25% oder mehr (Gew./Gew.) Humulus-Extrakt, z.B. Hopfenblüten-Extrakt.

In einer bevorzugten Ausführungsform umfasst die erfindungsgemäße Zusammensetzung einen Extrakt aus Grünkohl, vorzugsweise aus Grünkohlblättern, und einen Extrakt aus Baldrian und/oder Hopfen. In einer besonders bevorzugten Ausführungsform umfasst die erfindungsgemäße Zusammensetzung einen Extrakt aus Grünkohl, vorzugsweise aus Grünkohlblättern, und einen Extrakt aus Baldrianwurzeln und/oder Hopfenblüten.

Liegen die wirksamen pflanzlichen Bestandteile in den erfindungsgemäßen Zusammensetzungen als zerkleinerte oder unzerkleinerte Teile der entsprechenden Pflanzen vor, so liegt die bevorzugte Tagesmenge etwa 8 x bis etwa 15 x höher als bei Verabreichung eines Pflanzenextrakts.

Demgemäß wird die bevorzugte Tagesmenge von Teilen einer Grünkohl-Pflanze bei oralen oder rektalen Verabreichungsformen etwa 400-15000 mg betragen, vorzugsweise etwa 1000-10000 mg, 3000-8000 mg, und besonders bevorzugt etwa 4000-7000 mg, z.B. etwa 5500 mg. Die zu verabreichende Tagesmenge kann in einer Dosiseinheit oder in mehreren Dosiseinheiten, beispielsweise in 1, 2 oder 3 Dosiseinheiten verabreicht werden. In einer besonders bevorzugten Ausführungsform der Erfindung beziehen sich die obigen Mengenangaben auf Teile einer GrünkohlPflanze.

Die bevorzugte Tagesmenge von Teilen einer Pflanze der Gattung Valeriana und/oder der Gattung Humulus wird bei oralen oder rektalen Verabreichungsformen etwa 800-22500 mg betragen, vorzugsweise etwa 1000-20000 mg, etwa 3000-18000 mg, und besonders bevorzugt etwa 5000-16000 mg, z.B. etwa 10000 mg. Die zu verabreichende Tagesmenge kann in einer Dosiseinheit oder in mehreren Dosiseinheiten, beispielsweise in 1, 2 oder 3 Dosiseinheiten verabreicht werden. In einer besonders bevorzugten Ausführungsform der Erfindung beziehen sich die obigen Mengenangaben auf einen Teile einer Baldrianwurzel bzw. eines Hopfenzapfens.

Demgemäß umfassen die erfindungsgemäßen Zusammensetzungen vorzugsweise bezogen auf das Gesamtgewicht der Zusammensetzung:
- mindestens 1%, 2%, 3%, 4%, 5%, 6%, 7%, 8%, 9%, 10%, 12% 15%, 20%, 22% oder 25% oder mehr (Gew./Gew.) Teile einer Grünkohl-Pflanze, und
- mindestens 1%, 2%, 3%, 4%, 5%, 6%, 7%, 8%, 9%, 10%, 12% 15%, 20%, 22% oder 25% oder mehr (Gew./Gew.) Teile einer Pflanze der Gattung Valeriana, z.B. Teile einer Baldrian-Pflanze, und/oder
- mindestens 1%, 2%, 3%, 4%, 5%, 6%, 7%, 8%, 9%, 10%, 12% 15%, 20%, 22% oder 25% oder mehr (Gew./Gew.) Teile einer Pflanze der Gattung Humulus, z.B. Teile einer Hopfenblüte.

In einer bevorzugten Ausführungsform umfasst die erfindungsgemäße Zusammensetzung Pflanzenteile einer Grünkohlpflanze, vorzugsweise Teile von Grünkohlblättern, und Teile einer Baldrian- und/oder Hopfenpflanze. In einer besonders bevorzugten Ausführungsform umfasst die erfindungsgemäße Zusammensetzung Teile einer Grünkohlpflanze, vorzugsweise Teile aus Grünkohlblättern, und Teile aus Baldrianwurzeln und/oder Hopfenblüten.

Die nachstehenden Beispiele belegen, dass die kombinierte Gabe von Grünkohl-Extrakten und Valeriana-Extrakten oder Grünkohl-Extrakten und Humulus-Extrakten zu einer signifikanten Verstärkung von solchen elektrischen Impulsen im Gehirn von Versuchstieren führten, die allgemein mit einem Ruhezustand des Tiers assoziiert sind. Dies belegt, dass die kombinierte Gabe von Grünkohl-Extrakten und Valeriana-Extrakten oder Grünkohl-Extrakten und Humulus-Extrakten eine beruhigende und schlaffördernde Wirkung ausübt. Die Veränderung der im EEG beobachteten Muster war bei der kombinierten Gabe von Grünkohl-Extrakten und Valeriana-Extrakten oder Grünkohl-Extrakten und Humulus-Extrakten deutlich stärker als bei der getrennten Gabe der jeweiligen Extrakte.

Somit betrifft die Erfindung in einem weiteren Aspekt die Verwendung einer wie oben beschriebenen Extrakt-haltigen Zusammensetzung als Arzneimittel oder Beruhigungsmittel. Aufgrund der beruhigenden bzw. schlaffördernden Wirkung der erfindungsgemäßen, Extrakt-haltigen Zusammensetzungen eignen sich diese insbesondere für die Behandlung von Zuständen, die mit Schlaflosigkeit, Hyperaktivität und erhöhter Nervosität einhergehen.

In einem weiteren Aspekt betrifft die Erfindung die Verwendung einer wie oben beschriebenen Extrakt-haltigen Zusammensetzung in einem Verfahren zur Behandlung oder einem Verfahren zur Prophylaxe von Schlafstörungen, Aufmerksamkeitsdefizit-/Hyperaktivitätsstörung (ADHS) und Zuständen erhöhter Nervosität.

Die Erfindung stellt ferner ein Arzneimittel oder Nahrungsergänzungsmittel bereit, das eine wie oben beschriebene Extrakt-haltige Zusammensetzung umfasst. Das Arznei- oder Nahrungsergänzungsmittel ist vorzugsweise für die orale Verabreichung formuliert. Verfahren zur Herstellung eines Arznei- oder Nahrungsergänzungsmittels, bei denen eine wie oben beschriebene Extrakt-haltige Zusammensetzung oder deren aktive Bestandteile verwendet werden, d.h. ein Grünkohl-Extrakt und ein Valeriana-Extrakt und/oder Humulus-Extrakt, werden ebenfalls bereitgestellt.

Ein die erfindungsgemäße Zusammensetzung umfassendes Arzneimittel oder ein die erfindungsgemäße Zusammensetzung umfassendes Nahrungsergänzungsmittel kann in verschiedenen Darreichungsformen vorliegen. So kann das Arznei- oder Nahrungsergänzungsmittel z.B. für die orale, parenterale oder topische Darreichung formuliert sein. In einer besonders bevorzugten Ausführungsform ist das Arznei- oder Nahrungsergänzungsmittel für die orale Verabreichung formuliert.

Die Herstellung von Arzneimitteln ist im Stand der Technik hinreichend bekannt, und es sind zahlreiche verschiedene Verfahren zur Herstellung solcher Arzneimittel beschrieben. Derartige Verfahren sowie geeignete Hilfsstoffe und Träger werden beispielsweise in "Remington: The Science and Practice of Pharmacy", Lippincott Williams & Wilkins; 21. Auflage (2005) oder in "Mitra, Lee & Cheng: Advanced Drug Delivery", John Wiley & Sons,; 1. Auflage (2014), beschrieben. Die Arzneimittel können beispielsweise in Form von Granulaten, Pulvern, Tabletten, Kapseln, Sirup, Suppositorien, Injektionen, Emulsionen, Suspensionen oder Lösungen vorliegen. Die Arzneimittel können für verschiedene Verabreichungsarten formuliert sein, beispielsweise für die orale, parenterale, topische, bukkale, sublinguale, transmukosale, rektale, subkutane, intrathekale, intravenöse, intramuskuläre, intraperitoneale, nasale, intraokulare oder intraventrikuläre Verabreichung. Die Formulierung als orale, parenterale oder topische Zusammensetzung ist besonders bevorzugt. In einer besonders bevorzugten Ausführungsform ist die Zusammensetzung für die orale Verabreichung formuliert. Die pharmazeutischen Zusammensetzungen können auch als Retardmittel formuliert werden.

Für die orale, bukkale und sublinguale Verabreichung werden in der Regel feste Formulierungen, wie z.B. Pulver, Suspensionen, Granulate, Tabletten, Pillen, Kapseln und Gelcaps verwendet. Diese können beispielsweise hergestellt werden, indem man die wirksamen Bestandteile (Grünkohl-Extrakt und Humulus-Extrakt und/oder Valeriana-Extrakt) mit mindestens einem Additiv oder mit mindestens einem Hilfsstoff vermischt. Derartige Hilfsstoffe und Träger werden beispielsweise in "Remington: The Science and Practice of Pharmacy", Lippincott Williams & Wilkins; 21. Auflage (2005) beschrieben. Als Additive oder Hilfsstoffe können beispielsweise mikrokristalline Cellulose, Methylcellulose, Hydroxypropyl-Methylcellulose, Casein, Albumin, Mannit, Dextran, Saccharose, Laktose, Sorbitol, Stärke, Agar, Alginate, Pectine, Kollagen, Glyceride oder Gelatine verwendet werden. Ferner können Arzneimittel für die orale Verabreichung Antioxidantien (z.B. Ascorbinsäure, Tocopherol oder Cystein), Gleitmittel (z.B. Magnesiumstearat), Mittel zur Konservierung (z.B. Paraben oder Sorbinsäure), Sprengmittel, Bindemittel, Verdicker, Geschmacksstoffe, Farbstoffe und ähnliche Substanzen umfassen.

Flüssige Formulierungen der erfindungsgemäßen Arzneimittel, die sich für die orale Verabreichung eignen, können beispielsweise als Emulsion, Sirup, Suspension oder Lösungen vorliegen. Diese Formulierungen können unter Verwendung einer sterilen Flüssigkeit als Träger (z.B. Öl, Wasser, Alkohol oder Kombinationen derselben) in Form von flüssigen Suspensionen oder Lösungen hergestellt werden. Für die orale oder parenterale Verabreichung können pharmazeutisch geeignete Tenside, Suspensionsmittel, Öle oder Emulgatoren zugefügt werden. Geeignete Öle umfassen z.B. Olivenöl, Sesamöl, Erdnussöl, Rapsöl und Maisöl. Geeignete Alkohole umfassen Ethanol, Isopropyl-Alkohol, Hexadecyl-Alkohol, Glycerin und Propylenglykol. Suspensionen können ferner Fettsäureester, wie Ethyloleat, Isopropylmyristat, Fettsäureglyceride und acetylierte Fettsäureglyceride umfassen. Ferner werden Suspensionen häufig auch mit Substanzen wie Mineralöl oder Petrolatum versetzt.

Ebenfalls offenbart wird die Verwendung eines Grünkohl-Extrakts zur Verstärkung der Wirkung eines schlaffördernden Mittels. Vorzugsweise handelt es sich bei dem schlaffördernden Mittel um einen Valeriana-Extrakt, wobei ein Baldrianwurzel-Extrakt besonders bevorzugt ist.

Gezeigt wird ferner die Verwendung eines Grünkohl-Extrakts zur Verstärkung der Wirkung eines schlaffördernden Mittels. Vorzugsweise handelt es sich bei dem schlaffördernden Mittel um einen Humulus-Extrakt, wobei ein Humulusblüten-Extrakt besonders bevorzugt ist.

### KURZE BESCHREIBUNG DER FIGUREN

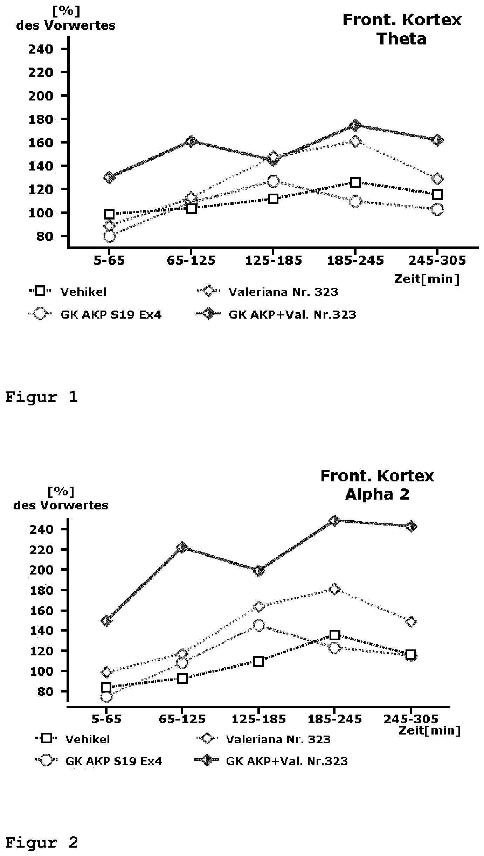

Figur 1 zeigt den zeitlichen Verlauf der Theta-Wellen über einen Zeitraum von 5 Stunden nach Verabreichung der einzelnen Extrakte (80 mg/kg Baldrianwurzelextrakt (Valeriana Nr. 323); 50 mg/kg Grünkohlextrakt (GK AKP S19 Ex4)) im Vergleich mit der Kombination von Baldrianwurzelextrakt und Grünkohlextrakt (80 mg/kg + 50 mg/kg) bei der Ratte. Die y-Achse zeigt die Leistungsdichte in % der Änderung vom Wert, der 45 Minuten vor der Verabreichung der Extrakte gemessen wurde. Es ist zu erkennen, dass bei alleiniger Gabe der beiden Extrakte diese nach einer Stunde noch keine Wirkung zeigen. In Kombination sind die Extrakte hingegen bereits nach einer Stunde wirksam. Die synergistische Wirkung ist überadditiv, insbesondere während der ersten beiden Stunden nach Verabreichung.
Figur 2 zeigt den zeitlichen Verlauf der Alpha 2-Wellen über einen Zeitraum von 5 Stunden nach Verabreichung der einzelnen Extrakte (80 mg/kg Baldrianwurzelextrakt; 50 mg/kg Grünkohlextrakt) im Vergleich mit der Kombination von Baldrianwurzelextrakt und Grünkohlextrakt (80 mg/kg + 50 mg/kg) bei der Ratte. Die y-Achse zeigt die Leistungsdichte in % der Änderung vom Wert, der 45 Minuten vor der Verabreichung der Extrakte gemessen wurde. Wie bereits für die Theta-Wellen beschrieben, entwickelt sich bereits eine Stunde nach oraler Gabe eine überadditive Wirkung.

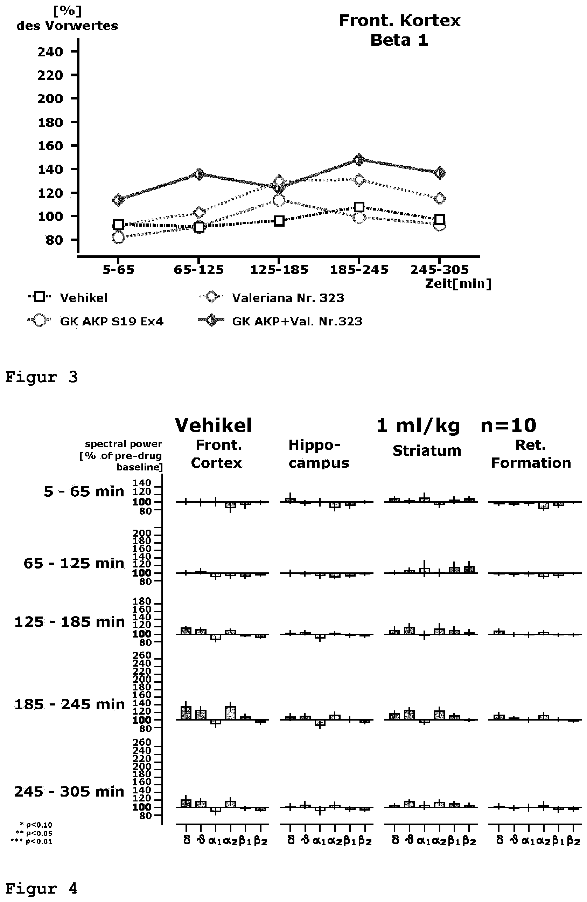

Figur 3 zeigt den zeitlichen Verlauf der Beta 1-Wellen über einen Zeitraum von 5 Stunden nach Verabreichung der einzelnen Extrakte (50 mg/kg Baldrianwurzelextrakt; 50 mg/kg Grünkohlextrakt) im Vergleich mit der Kombination von Baldrianwurzelextrakt und Grünkohlextrakt (80 mg/kg + 50 mg/kg) bei der Ratte. Die y-Achse zeigt die Leistungsdichte in % der Änderung vom Wert, der 45 Minuten vor der Verabreichung der Extrakte gemessen wurde. Wie bereits für die Theta- und Alpha 2-Wellen beschrieben, entwickelt sich bereits eine Stunde nach oraler Gabe eine überadditive Wirkung.
Figur 4 zeigt den zeitlichen Verlauf der elektrischen Leistung aller Frequenzbereiche über einen Zeitraum von 5 Stunden nach Verabreichung von Salzlösung (Vehikel) in den folgenden vier Hirnregionen: frontaler Kortex, Hippocampus, Striatum und Formatio reticularis. Die sechs Frequenzbereiche delta (δ), theta (θ), alpha1(α1), alpha2 (α2), beta1 (β1) und beta2 (β2) sind auf der Abszisse dokumentiert.

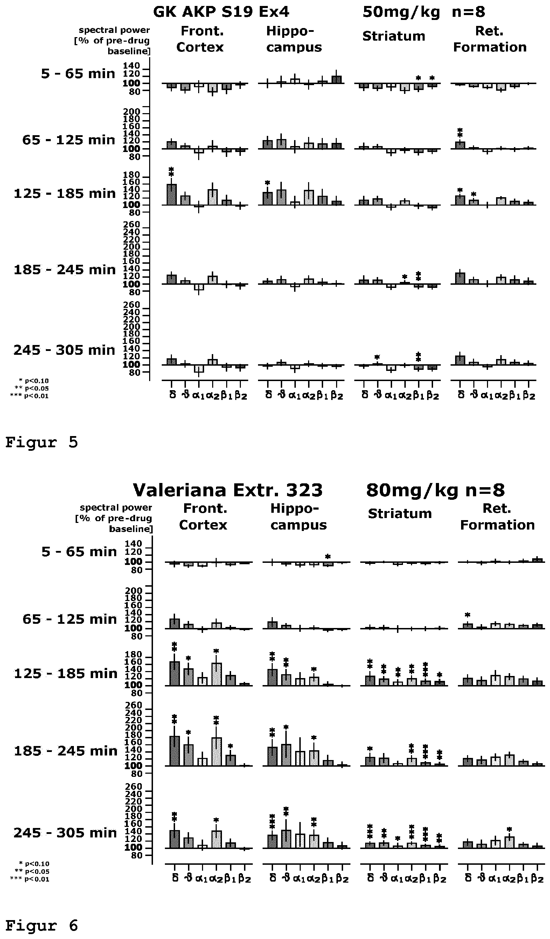

Figur 5 zeigt den zeitlichen Verlauf der elektrischen Leistung aller Frequenzbereiche über einen Zeitraum von 5 Stunden nach Verabreichung von 50 mg/kg Grünkohlextrakt allein in den folgenden vier Hirnregionen: frontaler Kortex, Hippocampus, Striatum und Formatio reticularis. Die sechs Frequenzbereiche delta (δ), theta (θ), alpha1(α1), alpha2 (α2), beta1 (β1) und beta2 (β2) sind auf der Abszisse dokumentiert. Die statistische Signifikanz im Vergleich zur Kontrolle (Vehikel) ist durch Sterne dokumentiert: *=p<0.10; **=p<0.05; ***=p<0.01.
Figur 6 zeigt den zeitlichen Verlauf der elektrischen Leistung aller Frequenzbereiche über einen Zeitraum von 5 Stunden nach Verabreichung von 80 mg/kg Baldrianwurzelextrakt allein in den folgenden vier Hirnregionen: frontaler Kortex, Hippocampus, Striatum und Formatio reticularis. Die sechs Frequenzbereiche delta (δ), theta (θ), alpha1(α1), alpha2 (α2), beta1 (β1) und beta2 (β2) sind auf der Abszisse dokumentiert. Die statistische Signifikanz im Vergleich zur Kontrolle (Vehikel) ist durch Sterne dokumentiert: *=p<0.10; **=p<0.05; ***=p<0.01.

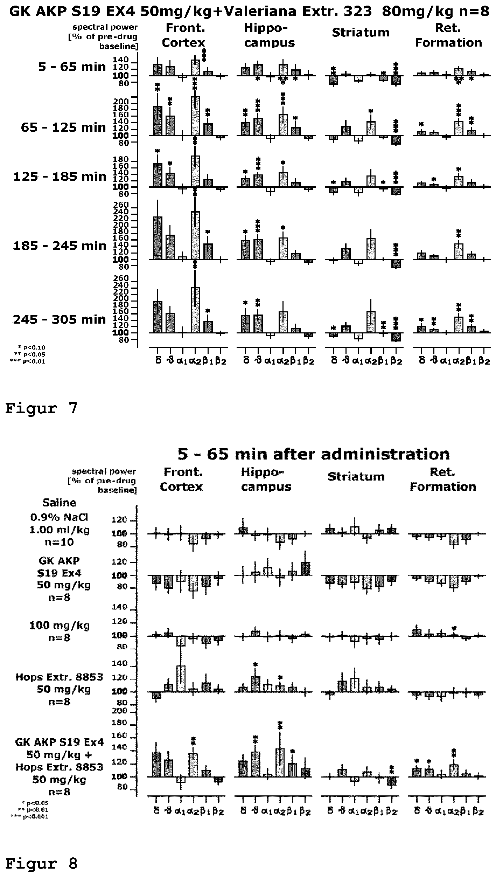

Figur 7 zeigt den zeitlichen Verlauf der elektrischen Leistung aller Frequenzbereiche über einen Zeitraum von 5 Stunden nach Verabreichung der Kombination von 80 mg/kg Baldrianwurzelextrakt und 50 mg/kg Grünkohlextrakt in den folgenden vier Hirnregionen: frontaler Kortex, Hippocampus, Striatum und Formatio reticularis. Die sechs Frequenzbereiche delta (δ), theta (θ), alpha1(α1), alpha2 (α2), beta1 (β1) und beta2 (β2) sind auf der Abszisse dokumentiert. Die statistische Signifikanz im Vergleich zur Kontrolle (Vehikel) ist durch Sterne dokumentiert: *=p<0.10; **=p<0.05; ***=p<0.01.

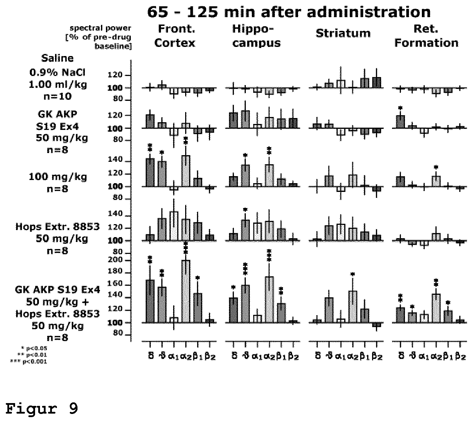

Figuren 8 und 9 zeigen den zeitlichen Verlauf der Theta-Wellen über einen Zeitraum von 1 bis 2 Stunden nach Verabreichung der einzelnen Extrakte (50 mg/kg Humulusextrakt (x); 50 mg/kg Grünkohlextrakt (GK AKP S19 Ex4)) im Vergleich mit der Kombination von Humlusextrakt und Grünkohlextrakt (50 mg/kg + 50 mg/kg) bei der Ratte. Die y-Achse zeigt die Leistungsdichte in % der Änderung vom Wert, der 45 Minuten vor der Verabreichung der Extrakte gemessen wurde. Es ist zu erkennen, dass bei alleiniger Gabe der beiden Extrakte diese nach einer Stunde noch keine signifikante Wirkung zeigen. In Kombination sind die Extrakte hingegen bereits nach einer Stunde signifikant wirksam. Die synergistische Wirkung ist überadditiv, insbesondere während der ersten beiden Stunden nach Verabreichung.

### BEISPIELE

Die nachfolgenden Beispiele beschreiben die physiologische Wirkung der erfindungsgemäßen Zusammensetzungen:

### Beispiel 1: Herstellung eines Grünkohlextrakts

Zur Herstellung eines Grünkohlextrakts, der im Rahmen der nachfolgenden Tierversuche verwendet werden kann, wurden 6 Kilogramm Blätter von *Brassica oleracea* convar. *acephala* var. *sabellica* (Grünkohl) mit einem Cutter zerkleinert. Der pH-Wert der resultierenden Maische wurde mit Ascorbinsäure auf einen Wert von 5 eingestellt. Der enzymatische Aufschluss der Zellen erfolgte mit den Enzymen Cellulase (0,025 ml Vegazym HC, 11000 Units/ml, Fa. Erbslöh) und Pektinase (0,025 ml Vegazym P, 40 Units/ml, Fa. Erbslöh). Die Enzyme wurden bei 37°C für 4 Stunden inkubiert. Der Ansatz wurde anschließend zentrifugiert.

Die Anreicherung der Polyphenole und Flavonoide erfolgte wie von Will et al. (2006, LWT - Food Science and Technology, 40 (8): 1344-1351) beschrieben. Der aus der Zentrifugation erhaltene Überstand wurde auf eine Adsorbersäule (XAD 16HD, Rohm & Haas, Frankfurt, Deutschland) geladen. Durch Spülen mit demineralisiertem Wasser wurden Salze, Aminosäuren, Peptide, Zucker und andere hydrophile Substanzen entfernt. Die Polyphenole wurden durch Elution mit Ethanol (95%) angereichert. Die nachfolgende Gefriertrocknung der eluierten Fraktionen liefert ein Pulver (ca. 80 g), das für nachfolgende Studien verwendet wurde.

### Beispiel 2: Tele-Stereo-EEG an der Ratte

Zur Messung von beruhigenden bzw. schlaffördernden Wirkungen von Kombinationen aus Grünkohlextrakt und Baldrianwurzelextrakt wurden die Veränderungen der EEG-Frequenzen nach oraler Gabe der Extrakte und nach Gabe einer Salzlösung als Negativkontrolle bestimmt. Die Untersuchungen wurden analog der Methode durchgeführt, die von Dimpfel beschrieben wurde (Dimpfel W (2003) Preclinical data base of pharmaco-specific rat EEG fingerprints (Tele-Stereo-EEG). Eur J Med Res (2003) 8: 199-207). Es wurde der Grünkohlextrakt aus Beispiel 1 sowie ein kommerziell erhältlicher Baldrianwurzelextrakt (Valerianae extractum Hydroalcoholicum siccum, Item Nr. 0199332, Finzelberg GmbH & Co KG, Andernach, Deutschland) verwendet.

Für die Messungen wurden 8 männlichen erwachsenen Fischer-344 Ratten (Tag-Nacht konvertiert) im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippocampus, Striatum und Formatio reticularis abgeleiteten Feldpotentiale. Die Signale wurden auf einem Computer-System (Software "EEG-Analyse", Betriebssystem OS Science, Laborrechner "LabTeam" der Firma MediSyst, Linden, DE) in Echtzeit einer Fast-Fourier-Transformation unterworfen und die Leistungsdichtespektren jeweils über 60 Minuten gemittelt. Die Unterteilung der Spektren in 6 verschiedene Frequenzbereiche erlaubte die Erfassung pharmakospezifischer Veränderungen in Bezug auf die jeweils vor Applikation gemessenen Referenzwerte innerhalb dieser Frequenzbänder.

Als Testpräparate wurden entweder Salzlösung (Negativkontrolle) oder Grünkohlextrakt bzw. Baldrianwurzelextrakt verabreicht. Die pflanzlichen Extrakte wurden jeweils getrennt voneinander und in Kombination appliziert. Die Testpräparate wurden 45 Minuten nach Beginn der Messungen (Referenzwert) oral an die Tiere verabreicht. Fünf Minuten nach der Verabreichung wurden die Messungen der EEG-Frequenzen wieder aufgenommen und für einen Zeitraum von mindestens 5 Stunden fortgeführt. Die Messwerte wurden kontinuierlich analysiert und in 60-minütige Perioden zugeordnet. Der statistische Vergleich der Versuche zu den Ergebnissen, die nach Gabe von Salzlösung gemessen wurde, erfolgte nach Wilcoxon, Mann and Whitney auf der Basis der Veränderungen innerhalb der einzelnen Frequenzbänder in allen Hirnregionen als Variablen.

### Ergebnisse:

Die Ergebnisse sind in den Figuren 1-8 dargestellt. Es ist zu erkennen, dass die Verabreichung von Salzlösung kaum zu Veränderungen der elektrischen Leistung (µV²) im Vergleich zu den Vorphasenwerten führte (siehe Figuren 1-4 und 8). Die getrennte Verabreichung von Baldrianwurzelextrakt oder Grünkohlextrakt führte zu schwachen, gerade messbaren Veränderungen der spektralen Leistung, die erst ab der zweiten Stunde nach Verabreichung erkennbar wurden (siehe Figuren 1, 2, 3 sowie 5 und 6) .

Die Verabreichung von Grünkohlextrakt in Kombination mit Baldrianwurzelextrakt dagegen führte bereits innerhalb der ersten Stunde vor allem im Kortex zu einer stabilen Veränderung der Leistungsdichte. In der dritten bis fünften Stunde nach Verabreichung der Extrakte war dieser Effekt sehr stark (siehe Figuren 1, 2, 3 sowie 7).

Eine ähnliche Wirkung ist bei der kombinierten Gabe von Grünkohl- und Hopfenextrakt zu sehen (Fig. 8 und 9). Die getrennte Verabreichung von Hopfenzapfenextrakt (getrockneter Extrakt 8853, Artikel-Nr. 255592, Plantextrakt GmbH & Co. KG, Vestenbergsgreuth, Deutschland) oder Grünkohlextrakt führte zu schwachen, gerade messbaren Veränderungen der spektralen Leistung. Bereits nach einer Stunde nach Gabe der Extraktkombination konnten jedoch signifikante Veränderungen gemessen.

Zusammenfassend lässt sich feststellen, dass die orale Verabreichung einer Kombination von Baldrianwurzelextrakt und Grünkohlextrakt oder von Hopfenblütenextrakt und Grünkohlextrakt zu deutlichen und statistisch signifikanten Veränderungen der elektrischen Hirnaktivität bei den Testtieren führte, die denen während des Schlafes entsprachen. Dies war besonders überraschend, da die Präparate am Morgen, d.h. nach der Nachtruhe (inaktive Phase bei Ratten), verabreicht wurden.

## Patentansprüche

1. Zusammensetzung, umfassend:
(a) mindestens einen Extrakt aus einer Pflanze der Gattung Brassica oder Teile einer Pflanze der Gattung Brassica, wobei die Pflanze der Gattung Brassica Grünkohl ist, und
(b1) mindestens einen Extrakt aus einer Pflanze der Gattung Valeriana oder Teile einer Pflanze der Gattung Valeriana, und/oder
(b2) mindestens einen Extrakt aus einer Pflanze der Gattung Humulus oder Teile einer Pflanze der Gattung Humulus.

2. Zusammensetzung nach Anspruch 1, wobei (i) der Extrakt der Pflanze der Gattung Brassica aus den Blättern der Brassica-Pflanze gewonnen wurde oder (ii) die Teile der Pflanze der Gattung Brassica die Blätter der Pflanze sind.

3. Zusammensetzung nach einem der Ansprüche 1-2, wobei die Pflanze der Gattung Valeriana ausgewählt ist aus der Gruppe bestehend aus V. altaica, V. amurensis, V. arborea, V. arizonica, V. barbulata, V. californica, V. capitata, V. celtica L., V. columbiana, V. dioica L., V. elongata, V. kassarica, V. montana L., V. occidentalis, V. officinalis, V. procurrens, V. pyrenaica L., V. saliunca, V. saxatilis L., V. tripteris L., V. wallrothii oder V. supina, wobei echter Baldrian (V. officinalis) besonders bevorzugt ist.

4. Zusammensetzung nach einem der Ansprüche 1-3, wobei (i) der Extrakt der Pflanze der Gattung Valeriana aus den Wurzeln der Valeriana Pflanze gewonnen wurde oder (ii) die Teile der Pflanze der Gattung Valeriana die Wurzeln der Pflanze sind.

5. Zusammensetzung nach einem der Ansprüche 1-4, wobei die Pflanze der Gattung Humulus ausgewählt ist aus der Gruppe bestehend aus H. lupulus L. (echtem Hopfen), H. scandens (Lour.) und H. yunnanensis Hu, wobei H. lupulus L. (echter Hopfen) besonders bevorzugt ist.

6. Zusammensetzung nach Anspruch 5, wobei (i) der Extrakt der Pflanze der Gattung Humulus aus den Blüten der Humulus Pflanze gewonnen wurde oder (ii) die Teile der Pflanze der Gattung Humulus die Blüten der Pflanze sind.

7. Zusammensetzung nach einem der Ansprüche 1-6 zur Verwendung als Arzneimittel oder Beruhigungsmittel.

8. Zusammensetzung nach einem der Ansprüche 1-6 zur Verwendung in einem Verfahren zur Behandlung oder Prophylaxe von Schlafstörungen, Aufmerksamkeitsdefizit-/Hyperaktivitätsstörung (ADHS), Zuständen erhöhter Nervosität oder zur Verbesserung der kognitiven Fähigkeiten, insbesondere zur Erhöhung der Konzentrationsfähigkeit.

9. Zusammensetzung zur Verwendung in einem Verfahren nach Anspruch 8, wobei das Verfahren die Verabreichung des Extrakts aus einer Pflanze der Gattung Brassica in einer Menge von 50-1000 mg pro Tag umfasst, wobei die Pflanze der Gattung Brassica Grünkohl ist.

10. Zusammensetzung zur Verwendung in einem Verfahren nach einem der Ansprüche 8-9, wobei das Verfahren die Verabreichung des Extrakts aus einer Pflanze der Gattung Valeriana und/oder Humulus in einer Menge von 100-1500 mg pro Tag umfasst.

11. Arzneimittel, Nahrungsergänzungsmittel oder Lebensmittel, das eine Zusammensetzung nach einem der Ansprüche 1-9 umfasst.

12. Arzneimittel, Nahrungsergänzungsmittel oder Lebensmittel nach Anspruch 11, das für die orale Verabreichung formuliert ist.

## Claims

1. Composition comprising:
(a) at least one extract of a plant of the genus Brassica or parts of a plant of the genus Brassica, wherein said plant of the genus Brassica is curly kale, and
(b1) at least one extract of a plant of the genus Valeriana or parts of a plant of the genus Valeriana, and/or
(b2) at least one extract of a plant of the genus Humulus or parts of a plant of the genus Humulus.

2. Composition according to claim 1, wherein (i) the extract of the plant of the genus Brassica has been obtained from the leaves of the Brassica plant, or (ii) the parts of the plant of the genus Brassica are the leaves of the plant.

3. Composition according to any of claims 1-2, wherein the plant of the genus Valeriana is selected from the group consisting of V. altaica, V. amurensis, V. arborea, V. arizonica, V. barbulata, V. californica, V. capitata, V. celtica L., V. columbiana, V. dioica L., V. elongata, V. kassarica, V. montana L., V. occidentalis, V. officinalis, V. procurrens, V. pyrenaica L., V. saliunca, V. saxatilis L., V. tripteris L., V. wallrothii or V. supina, wherein true valerian (V. officinalis) is particularly preferred.

4. Composition according to any of claims 1-3, wherein (i) the extract of the plant of the genus Valeriana has been obtained from the roots of the Valeriana plant or (ii) the parts of the plant of the genus Valeriana are the roots of the plant.

5. Composition according to any of claims 1-4, wherein the plant of the genus Humulus is selected from the group consisting of H. lupulus L. (true hops), H. scandens (Lour.) and H. yunnanensis Hu, wherein H. lupulus L (real hops) is particularly preferred.

6. Composition according to claim 5, wherein (i) the extract of the plant of the genus Humulus has been obtained from the blossoms of the Humulus plant, or (ii) the parts of the plant of the genus Humulus are the blossoms of the plant.

7. Composition according to any of claims 1-6 for use as a medicinal product or sedative.

8. Composition according to any of claims 1-6 for use in a method of treatment or prophylaxis of sleep disorders, attention deficit/hyperactivity disorder (ADHD), conditions of increased nervousness or for the improvement of cognitive abilities, in particular for increasing the ability to concentrate.

9. Composition for use in a procedure according to claim 8, wherein the procedure comprises administering the extract of a plant of the genus Brassica in an amount of 50-1000 mg per day, wherein said plant of the genus Brassica is curly kale.

10. Composition for use in a procedure according to any of claims 8-9, wherein the procedure comprises administering the extract of a plant of the genus Valeriana and/or Humulus in an amount of 100-1500 mg per day.

11. Medicinal product, dietary supplement or food product comprising a composition according to any of claims 1-9.

12. Medicinal product, dietary supplement or food product according to claim 11, which is formulated for oral administration.

## Revendications

1. Composition comprenant :
a) au moins un extrait d'un végétal du genre Brassica ou de parties d'un végétal du genre Brassica, lequel végétal du genre Brassica est le chou frisé dit « chou kalé »,
b1) et au moins un extrait d'un végétal du genre Valeriana ou de parties d'un végétal du genre Valeriana,
b2) et/ou au moins un extrait d'un végétal du genre Humulus ou de parties d'un végétal du genre Humulus.

2. Composition conforme à la revendication 1, pour laquelle
i) l'extrait du végétal du genre Brassica a été obtenu à partir de feuilles du végétal Brassica,
ii) ou les parties du végétal du genre Brassica sont des feuilles du végétal.

3. Composition conforme à l'une des revendications 1 et 2, pour laquelle le végétal du genre Valeriana est choisi dans l'ensemble formé par les suivants : V. altaica, V. amurensis, V. arborea, V. arizonica, V. barbulata, V. californica, V. capitata, V. celtica L., V. columbiana, V. dioica L., V. elongata, V. kassarica, V. montana L., V. occidentalis, V. officinalis, V. procurrens, V. pyrenaica L., V. saliunca, V. saxatilis L., V. tripteris L., V. wallrothii et V. supina, étant entendu que la vraie valériane, V. officinalis, est particulièrement préférée.

4. Composition conforme à l'une des revendications 1 à 3, pour laquelle
i) l'extrait du végétal du genre Valeriana a été obtenu à partir de racines du végétal Valeriana,
ii) ou les parties du végétal du genre Valeriana sont des racines du végétal.

5. Composition conforme à l'une des revendications 1 à 4, pour laquelle le végétal du genre Humulus est choisi dans l'ensemble formé par les suivants : H. lupulus L. (vrai houblon), H. scandens (Lour.) et H. yunnanensis Hu, étant entendu que le vrai houblon, H. lupulus L., est particulièrement préféré.

6. Composition conforme à la revendication 5, pour laquelle
i) l'extrait du végétal du genre Humulus a été obtenu à partir de fleurs du végétal Humulus,
ii) ou les parties du végétal du genre Humulus sont des fleurs du végétal.

7. Composition conforme à l'une des revendications 1 à 6, pour utilisation en tant que produit médicinal ou sédatif.

8. Composition conforme à l'une des revendications 1 à 6, pour utilisation dans un procédé de traitement ou de prophylaxie de troubles du sommeil, d'un trouble de déficit d'attention avec hyperactivité (TDAH), ou d'états de grande nervosité, ou pour l'amélioration de capacités cognitives, en particulier pour augmenter la capacité de concentration.

9. Composition pour utilisation dans un procédé conforme à la revendication 8, lequel procédé comporte le fait d'administrer l'extrait d'un végétal du genre Brassica en une dose de 50 à 1000 mg par jour, le végétal du genre Brassica étant le chou kalé.

10. Composition pour utilisation dans un procédé conforme à l'une des revendications 8 et 9, lequel procédé comporte le fait d'administrer l'extrait d'un végétal du genre Valeriana et/ou Humulus en une dose de 100 à 1500 mg par jour.

11. Produit médicinal, supplément diététique ou produit alimentaire comprenant une composition conforme à l'une des revendications 1 à 9.

12. Produit médicinal, supplément diététique ou produit alimentaire conforme à la revendication 11, formulé pour être administré par voie orale.
